(19) Europäisches Patentamt
European Patent Office
Office européen des brevets

(11) Veröffentlichungsnummer: **0 022 955**
**B1**

(12) # EUROPÄISCHE PATENTSCHRIFT

(45) Veröffentlichungstag der Patentschrift: **08.06.83**

(51) Int. Cl.³: **C 07 C 49/203,** C 07 C 45/68

(21) Anmeldenummer: **80103719.3**

(22) Anmeldetag: **01.07.80**

(54) **Verbessertes Verfahren zur Herstellung von höheren ungesättigten Ketonen.**

(30) Priorität: **18.07.79 DE 2928944**

(43) Veröffentlichungstag der Anmeldung: **28.01.81 Patentblatt 81/4**

(45) Bekanntmachung des Hinweises auf die Patenterteilung: **08.06.83 Patentblatt 83/23**

(84) Benannte Vertragsstaaten: **BE CH DE FR GB LI NL**

(56) Entgegenhaltungen:
DE-B-1 053 498
DE-B-1 068 696
DE-B-1 793 445
FR-A-2 371 411
GB-A- 822 572
GB-A- 951 927
GB-A-1 463 184

(73) Patentinhaber: **BASF Aktiengesellschaft, *Carl-Bosch-Strasse 38, D-6700 Ludwigshafen (DE)***

(72) Erfinder: **Nissen, Axel, Dr., Panoramastrasse 51, *D-6906 Leimen (DE)***
Erfinder: **Kaibel, Gerd, Robert-Bosch-Strasse 4, D-6840 Lampertheim (DE)**
Erfinder: **Woerz, Otto, Bruesseler Ring 51, D-6700 Ludwigshafen (DE)**
Erfinder: **Arnold, Lothar, Dr., Hausackerweg 11, D-6900 Heidelberg (DE)**
Erfinder: **Braun, Manfred, Friedensstrasse 39, D-6704 Mutterstadt (DE)**
Erfinder: **Rebafka, Walter, Dr., Schuetzenstrasse 4, D-6901 Eppelheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 022 955 B1

ACTORUM AG

Verbessertes Verfahren zur Herstellung von höheren ungesättigten Ketonen

Die vorliegende Erfindung betrifft ein verbessertes Verfahren zur Herstellung von höheren ungesättigten Ketonen durch Umsetzen von $\alpha,\beta$-ungesättigten Alkoholen mit Acetessigsäurealkylestern bei erhöhter Temperatur in Gegenwart von organischen Aluminiumverbindungen unter Abspaltung und Entfernung des aus dem Acetessigester stammenden Alkohols.

Diese Reaktion ist, sieht man von den erfindungsgemässen Verbesserungen ab, in ihren wesentlichen Merkmalen bereits bekannt. Ursprünglich wurde eine derartige Umsetzung zwischen einem ungesättigten Alkohol und einem Acetessigsäurealkylester von Carroll [J. Chem. Soc. (London) *1941*, Seiten 507 bis 511] beschrieben. Eine neuere Verfahrensvorschrift für die Herstellung von 6,10,14-Trimethyl-5-pentadecen-2-on ist der franz. Patentschrift 1 219 166 zu entnehmen. Gemäss diesem Verfahren wird das gewünschte Keton in einer Reaktionszeit von etwa 10 Stunden in 77%iger Ausbeute erhalten.

Für eine technische Synthese unbefriedigend an diesem Verfahren sind sowohl die verhältnismässig langen Reaktionszeiten als auch die unzureichenden Ausbeuten. Die unzureichenden Ausbeuten sind besonders gravierend bei der Herstellung von höheren Ketonen, d.h. bei Verwendung von höheren Alkoholen der Formel II, da diese mit wachsender Kettenlänge teurer in ihrer Herstellung werden. Versucht man die Ausbeuten dadurch zu verbessern, dass man die billigere Komponente, hier den Acetessigsäurealkylester, im Überschuss verwendet, so kommt es leicht zur Bildung von Dehydrazetsäure als Nebenprodukt, die später aus dem gewünschten Produkt nur schwer wieder abzutrennen ist und ausserdem die Ablaufleitungen der bei der Reinigung verwendeten Kolonnen verstopfen kann.

Es sind eine Reihe weiterer Patentschriften bekannt, in denen die verschiedenen Varianten dieser sogenannten Carroll'schen Reaktion beschrieben werden. So heisst es z.B. in der DE-PS 1 068 696, dass die Mitverwendung eines Lösungsmittels vorteilhaft sein könnte. In der US-PS 2 795 617 bzw. der DE-AS 1 053 498 heisst es, dass die Mitverwendung von Lösungsmitteln «möglich, jedoch normalerweise weder notwendig noch erwünscht» sei. In allen Fällen, in denen die Mitverwendung von Lösungsmitteln erwähnt wird, werden hochsiedende Lösungsmittel genannt, deren Siedepunkte weit oberhalb der Reaktionstemperatur liegen, wie Decalin (Kp = 193°C), Tetralin (Kp = 207°C), Diphenyläther (Kp = 259°C), Siliconöl und hochsiedende Mineralöle.

Die Mitverwendung dieser hochsiedenden Lösungs- oder Verdünnungsmittel bringt jedoch im allgemeinen keine nennenswerte Verbesserung der Ausbeuten an Ketonen mit sich.

Es war daher die Aufgabe der Erfindung, dass oben erläuterte Verfahren so zu verbessern, dass die höheren ungesättigten Ketone, insbesondere die als Riechstoffe und als Zwischenprodukte für Isophytol begehrten Ketone wie Geranylaceton, Farnesylaceton, 6,10-Dimethyl-5-undecen-2-on und 6,10,14-Trimethyl-5-pentadecen-2-on in höheren Ausbeuten sowie in höheren Raum-Zeit-Ausbeuten hergestellt werden können.

Es wurde nun gefunden, dass sich die ungesättigten Ketone der allgemeinen Formel I

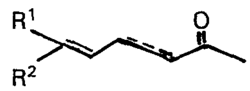

(I)

in der die gestrichelte Linie eine zusätzliche C-C-Bindung bedeuten kann, $R^1$ für eine Alkylgruppe mit 1 bis 4 C-Atomen, vorzugsweise $CH_3$ steht, und $R^2$ für eine gesättigte oder ungesättigte Alkyl-, Cycloalkyl- oder Cycloalkylalkylgruppe mit 4 bis 30 C-Atomen, oder vorzugsweise für eine Gruppe der allgemeinen Formel

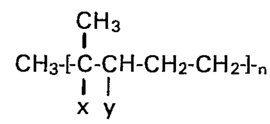

steht, in der n für eine ganze Zahl von 1 bis 6 steht und x und y entweder beide für H stehen, oder x für Methoxy und y für H stehen, oder aber x und y zusammen eine zusätzliche Bindung zwischen den x und y tragenden C-Atomen bedeuten, durch Umsetzen von ungesättigten Alkoholen der allgemeinen Formel II

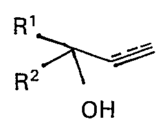

(II)

mit Acetessigsäurealkylestern der allgemeinen Formel III

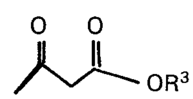

(III)

in der $R^3$ für Alkyl mit 1 bis 4 C-Atomen steht, bei erhöhter Temperatur in Gegenwart von 0,5 bis 5 Gew.-%, bezogen auf den umzusetzenden Acetessigsäurealkylester, an einer organischen Aluminiumverbindung der allgemeinen Formel

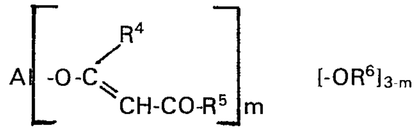

in der $R^4$ und $R^5$ Alkyl- oder Alkoxygruppen mit 1 bis 4 C-Atomen, vorzugsweise Methyl- oder Äthylgruppen bedeuten, $R^6$ für eine Alkylgruppe mit 1 bis 4 C-Atomen steht und m jeweils für die Zahlen 0, 1, 2 oder 3 steht oder einem Aluminiumtriaryloxylat unter Abspaltung und fortlaufender destillativer Entfernung eines Alkohols der allgemeinen Formel IV

$$R^3 - OH \qquad (IV)$$

in einem Reaktorsystem mit aufgesetzter Fraktionierkolonne mit wesentlich besseren Ausbeuten und Raum-Zeit-Ausbeuten erhält, wenn man

A. von einem ungesättigten Alkohol der Formel II ausgeht, dessen Siedepunkt höher liegt als der des verwendeten Acetessigsäurealkylesters;
B. die Umsetzung in Gegenwart von 0,5 bis 100 Gew.-%, bezogen auf den Alkohol der Formel II, einer Flüssigkeit (im folgenden als «Zwischensieder» bezeichnet) durchführt, die sich unter den Reaktionsbedingungen inert verhält und deren Siedepunkt zwischen dem des eingesetzten Acetessigesters der allgemeinen Formel III und dem des hieraus abzuspaltenden Alkohols der allgemeinen Formel IV liegt, oder aber dass man die Umsetzung in Gegenwart von 3-Methyl-1-buten-3-ol als Zwischensieder durchführt, wobei zusätzlich als erwünschte Nebenreaktion dessen Umsetzung mit dem Acetessigsäurealkylester zu 2-Methyl-2-hepten-6-on als weiterem Wertprodukt stattfindet, und
C. dafür sorgt, dass die Temperatur am unteren Ende der Fraktionierkolonne nicht mehr als 40°C, vorzugsweise 20°C, höher ist als der Siedepunkt des zugesetzten Zwischensieders beim jeweils verwendeten Druck.

Das erfindungsgemässe Verfahren gelingt mit besonderem Vorteil, wenn man als definitionsgemässe Zwischensieder ein aliphatisches Keton mit 4 bis 7 C-Atomen verwendet und wenn man als Acetessigsäurealkylester entweder Acetessigsäuremethylester, Acetessigsäureäthylester oder Acetessigsäureisopropylester verwendet. Es hat besondere Bedeutung für Ketone der allgemeinen Formel I, in der $R^1$ für $CH_3$ und $R^2$ für eine Gruppe der allgemeinen Formel

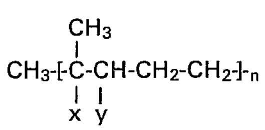

steht, in der n für eine ganze Zahl von 1 bis 6 steht und x und y entweder beide für H stehen, oder x für Methoxy und y für stehen, oder aber x und y zusammen eine zusätzliche Bindung zwischen x und y tragenden C-Atomen bedeuten.

Eine weitere besonders vorteilhafte Ausführungsform des erfindungsgemässen Verfahrens ist dadurch gekennzeichnet, dass man die Umsetzung in Gegenwart von 3-Methyl-1-buten-3-ol durchführt, wobei zusätzlich als erwünschte Nebenreaktion eine Umsetzung von 3-Methyl-1-buten-3-ol mit dem Acetessigsäurealkylester zu 2-Methyl-2-hepten-6-on als weiterem Wertprodukt stattfindet.

Wesentlich ist, dass die erfindungsgemäss erzielbaren hohen Ausbeuten bei nahezu quantitativem Umsatz, bezogen auf alle Einsatzkomponenten, erzielt werden und zwar auch dann, wenn kein Überschuss oder ein nur geringer Überschuss einer der Komponenten eingesetzt wird. Bemerkenswert ist auch, dass durch die erfindungsgemässen Massnahmen die Raum-Zeit-Ausbeuten um den Faktor 2 bis 3 gesteigert werden können.

Als Flüssigkeiten, die sich unter den Reaktionsbedingungen inert verhalten oder keine unerwünschten Nebenreaktionen eingehen können, sogenannte «Zwischensieder», kommen erfindungsgemäss solche Flüssigkeiten in Betracht, deren Siedepunkt zwischen dem des eingesetzten Acetessigesters der Formel III und dem des abgespaltenen Alkohols IV liegt, d.h. also Flüssigkeiten, die bei Normaldruck im allgemeinen zwischen 60 und 180°C, vorzugsweise 80 und 150°C und insbesondere zwischen 90 und 120°C sieden. Geeignet sind beispielsweise einige entsprechend siedenden Alkohole, Ester, Äther, halogenierte Kohlenwasserstoffe und aromatische Kohlenwasserstoffe, vorzugsweise jedoch aliphatische Ketone und von diesen solche mit 4 bis 7 C-Atomen. Genannt seien z.B. Isobutanol, 3-Methyl-1-buten-3-ol, Ameisensäurebutylester, Essigsäurebutylester, Toluol, o-Xylol, m-Xylol, p-Xylol, Äthylbenzol, 1,1,2-Trichloräthan, Trichlorfluoräthan, 1,4-Dioxan, Butan-2-on, 3-Methyl-butan-2-on, Pentan-3-on und Heptanon-(2), insbesondere Butan-2-on, 3-Methyl-butan-2-on und Pentan-3-on.

Die Zwischensieder sollten zweckmässigerweise mit dem während der Reaktion abgespaltenen Alkohol der Formel IV, z.B. Methanol, Äthanol und Isopropanol, keine Azeotrope bilden. Diese Bedingung ist jedoch lediglich für die Rückgewinnbarkeit des Zwischensieders von Bedeutung, nicht jedoch für die vorteilhafte Beeinflussung der Reaktion.

Die Zwischensieder verwendet man im allgemeinen in Mengen von 0,5 bis 100 Gew.%, bezogen auf den Alkohol der Formel II. Zweckmässigerweise wird er so gewählt, dass nur etwa 1 bis 20 Gew.%, bezogen auf den Alkohol der Formel II benötigt werden. Dies ist der Fall, wenn der Zwischensieder möglichst niedrig siedet, aber dennoch gut von dem Alkohol $R^3OH$ abtrennbar ist. Je dichter der Siedepunkt des Zwischensieders dagegen an den des Acetessigesters herankommt, desto mehr Zwischensieder wird benötigt, wodurch die Umsetzung verständlicherweise insgesamt ungünstiger wird.

Verwendet man als Zwischensieder eine Flüssigkeit, die sich unter den Reaktionsbedingungen nicht inert verhält sondern eine erwünschte Nebenreaktion eingeht, so muss man dem Reaktionsgemisch verständlicherweise ständig zusätzlich soviel Zwischensieder zusetzen, wie durch die Nebenreaktion dem Reaktionsgemisch entzogen wird. Als Beispiel für diese Variante des erfindungsgemässen Verfahrens sei die Umsetzung von Linalool, von 3,7-Dimethyl-1-octen-3-ol oder von 3,7,11-Trimethyl-1-dodecen-3-ol in Gegenwart von 3-Methyl-1-buten-3-ol genannt, bei der neben dem Geranylaceton, dem 6,10-Dimethyl-5-undecen-2-on bzw. dem 6,10,14-Trimethyl-5-pentadecen-2-on auch das als Zwischenprodukt für Vitamin A und Vitamin E begehrte 2-Methyl-2-hepten-6-on mit sehr guter Selektivität erhalten wird. Das 3-Methyl-1-buten-3-ol fungiert hierbei gleichzeitig als Zwischensieder wie auch als Reaktionspartner des Acetessigsäurealkylesters. Es wird daher insgesamt in solchen Mengen verwendet, wie es als Zwischensieder und als Reaktionspartner benötigt wird. Auf diese Weise lassen sich überraschenderweise die Vorteile der erfindungsgemässen Verfahrensverbesserung indirekt auch bei der Umsetzung von 3-Methyl-1-buten-3-ol mit Acetessigsäurealkylestern ausnützen, was sonst wegen des

niederen Siedepunktes dieses Alkohols nicht möglich gewesen wäre.

Im allgemeinen wird die erfindungsgemässe Umsetzung bei Normaldruck durchgeführt. Jedoch erfüllt ein für Normaldruck geeigneter Zwischensieder in aller Regel seine Funktion auch bei vermindertem Druck, d.h. er siedet meist auch in diesem Falle zwischen dem Alkohol IV und dem Acetessigester. In bestimmten Fällen kann es von Vorteil sein, unter vermindertem Druck zu arbeiten. Man muss dann den Druckbereich so wählen, dass die erforderliche Reaktionstemperatur (meist 120 bis 200°C, vorzugsweise 140 bis 170°C), eingehalten werden kann, zugleich aber eine gute Abtrennbarkeit des abgespaltenen Alkohols der Formel IV von den anderen im Reaktionsgemisch enthaltenen Komponenten und dem Zwischensieder gewährleistet ist.

Das Verfahren der Erfindung lässt sich prinzipiell auf alle bekannten Varianten der sogenannten Carroll'schen Reaktion anwenden, bei denen der eingesetzte ungesättigte Alkohol höher als der verwendete Acetessigsäurealkylester. Besondere Bedeutung besitzt das Verfahren jedoch für die Synthese solcher Ketone, die zur Herstellung von Isophytol benötigt werden, wie Geranylaceton und Farnesylaceton.

Besonders interessant ist das erfindungsgemässe Verfahren also für die Umsetzung von Alkoholen der allgemeinen Formel IIa

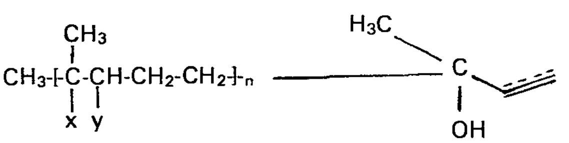

(IIa),

in der x, y und n die oben angegebene Bedeutung haben, wie beispielsweise Linalool, 3,7-Dimethyl-1-octen-3-ol, Nerolidol und 3,7,11-Trimethyl-1-dodecen-3-ol.

Die Reaktion gelingt prinzipiell mit beliebigen Acetessigsäurealkylestern, jedoch werden der Methylester, der Äthylester und der Isopropylester sowohl aus wirtschaftlichen als auch aus verfahrenstechnischen Gründen bevorzugt, da die hieraus abzuspaltenden Alkohole besonders niedrig sieden und so leicht aus dem Reaktionsgemisch entfernt werden können.

Als organische Aluminiumverbindungen kommen für das erfindungsgemässe Verfahren Verbindungen der allgemeinen Formel

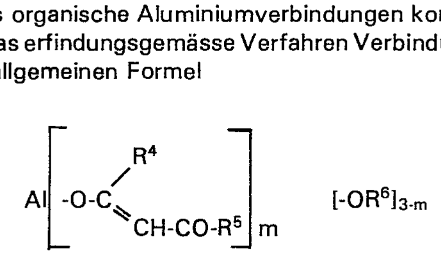

in der $R^4$ und $R^5$ Alkyl- oder Alkoxygruppen mit 1 bis 4 C-Atomen, vorzugsweise Methyl- oder Äthylgruppen bedeuten, $R^6$ für eine Alkylgruppe mit 1 bis 4 C-Atomen steht und m jeweils für die Zahlen 0, 1, 2 oder 3 steht, sowie Aluminiumtriaryloxylate in Betracht. Bei den ersten handelt es sich also um niedere Aluminiumtrialkoholate wie Aluminiumtrimethylat, -triäthylat, -triisopropylat, tri-sek.-butylat sowie um Verbindungen, die bei der Umsetzung der genannten Aluminiumtrialkoholate mit stöchiometrischen Mengen von Acetylacetonat, Alkylacetoacetat oder Alkylmalonat unter Alkoholabspaltung gebildet werden. Genannt seien beispielsweise Aluminiumtriacetoacetat, Aluminiumtriacetylacetonat, Aluminiummonoacetoacetat-diäthylat, Aluminiumdiacetoacetat-monoäthylat, Aluminiummonoacetoacetat-diisopropylat; Aluminiumdiacetoacetat-monoisopropylat. Bevorzugt verwendet man die Aluminiumtrialkoholate, insbesondere das Aluminiumtriisopropylat.

Als Aluminiumtriaryloxylate bezeichnen wir die Aluminiumsalze von aromatischen Hydroxyverbindungen, wie Aluminiumtriphenolat, Aluminiumtrikresolate, Aluminiumtrixylenolate, Aluminiumtrinaphtholate, deren Arylreste auch durch niedere Alkyl- oder Alkyloxygruppen, d.h. Alkyl- oder Alkyloxygruppen mit 1 bis 4 C-Atomen, Hydroxygruppen oder Phenyl substituiert sein können. Mit besonderem Vorteil verwendet man das relativ leicht zugängliche Aluminiumtriphenolat.

Die Menge der Aluminiumverbindung wird allgemein so bemessen, dass ihre Konzentration im Reaktionsgemisch 0,05 Gew.% Al nicht unterschreitet und zu Beginn der Reaktion 6 Gew.% Al nicht überschreitet. Bezogen auf umzusetzenden Acetessigsäurealkylester werden im allgemeinen 0,5 bis 5 Gew.% an der Aluminiumverbindung benötigt. Für das bevorzugt verwendete Aluminiumtriisopropylat sind z.B. Mengen von etwa 1 bis 3 Gew.%, bezogen auf den umzusetzenden Acetessigsäurealkylester.

Das erfindungsgemässe Verfahren kann sowohl diskontinuierlich als auch kontinuierlich durchgeführt werden.

Bei diskontinuierlicher Reaktionsführung wird zweckmässigerweise der ungesättigte Alkohol der Formel II vorgelegt, möglicherweise zusammen mit dem Zwischensieder und gegebenenfalls zusammen mit der als Katalysator verwendeten Aluminiumverbindung. Nach Einstellen der gewünschten Reaktionstemperatur wird der Acetessigester der Formel III innerhalb von etwa 2 bis 4 Stunden zugefahren. Wenn quantitativer Umsatz erwünscht ist, ist es vorteilhaft, das Reaktionsgemisch nach Beendigung des Zulaufs noch etwa 1 bis 2 Stunden bei der Reaktionstemperatur zu halten. Das Fortschreiten der Reaktion kann anhand der Kohlendioxidentwicklung und/oder anhand der Menge des vom Acetessigsäurealkylester abgespaltenen Alkanol verfolgt werden. Die Konzentration des Acetessigsäureesters im Reaktionsgemisch kann durch gaschromatographische Analyse bestimmt werden.

Es ist jedoch auch möglich, anstatt des Alkohols der Formel II den Acetessigester der Formel III oder Gemische beider, oder Gemische des Acetessigesters mit dem Zwischensieder vorzulegen und auch Gemische der Verbindungen II und III zuzufahren.

Als Reaktorsystem eignet sich eine übliche beheizte Destillationsblase mit aufgesetzter Fraktionierkolonne, beispielsweise einer Kolonne mit etwa 2 bis 40 theoretischen Böden. Die Temperatur auf den unteren 1 bis 3 theoretischen Böden der Kolonne sollte erfindungsgemäss möglichst nahe der Siedetemperatur des Zwischensieders bei dem jeweils angewandten Druck liegen. Werden am unteren Ende der Kolonne höhere Temperaturen eingestellt, so nimmt der Effekt des Verfahrens nur unwesentlich zu. Vermindert man die Temperatur am unteren Ende der Kolonne derart, dass sie sich dem Siedepunkt des Alkohol IV nähert, so nimmt der erfindungsgemässe Effekt stark ab. Die Temperaturen im Reaktionssystem werden durch dosierte Zugabe des Zwischensieders geregelt.

Bei kontinuierlicher Reaktionsführung kann als Reaktorsystem beispielsweise eine beheizte Kesselkaskade mit 1 bis 10, zweckmässigerweise 2 bis 4 Kesseln, verwendet werden. Hierbei sind die einzelnen Kessel durch einen Überlauf miteinander verbunden. Es kann jedem Kessel eine separate Kolonne aufgesetzt sein oder aber nur eine Kolonne für alle Kessel. Der Ablauf des untersten Bodens der Kolonne, der im wesentlichen den Zwischensieder enthält, wird zur Regelung der Temperatur auf die Kessel verteilt. Der Zulauf, bestehend aus den Verbindungen der Formeln II und III sowie gegebenenfalls dem Aluminiumkatalysator wird kontinuierlich in den ersten Kessel gefahren. Durch Zugabe des Zwischensieders wird die Temperatur reguliert.

Für das Einhalten der Temperaturen und des Drucks gilt das gleiche wie für die diskontinuierliche Fahrweise.

Mit Hilfe des erfindungsgemäss verbesserten Verfahrens ist es möglich, zahlreiche höhere Ketone, insbesondere solche Ketone, wie sie zur Herstellung von Isophytol und damit zur Herstellung von Vitamin E benötigt werden, wie Geranylaceton und Farnesylaceton, bei nahezu quantitativem Umsatz in sehr hohen Ausbeuten und Raumzeitausbeuten sowie hoher Reinheit herzustellen.

*Beispiel 1*

Herstellung von Geranylaceton

A. Als Apparatur wird ein 4-l-Kolben mit einer aufgesetzten Kolonne, die etwa 20 theoretische Böden aufweist, verwendet. Die Kolonne ist in regelmässigen Abständen mit Thermometern bestückt, so dass ausser im Sumpf und am Kopf der Kolonne etwa an jedem 3. bis 4. theoretischen Boden die Temperatur gemessen werden kann.

Ein Gemisch aus 616 g (4 Mol) Linalool, 10 g Aluminum-tri-sek.-butylat sowie 80 g 3-Methyl-butan-2-on (Siedepunkt: 95°C) wurde in dem Kolben vorgelegt und auf 165°C erhitzt. Zu diesem Gemisch wurden innerhalb von 4 Stunden 464 g (4 Mol) Acetessigsäuremethylester zugetropft und Methanol unter $CO_2$-Entwicklung über Kopf der Kolonne abdestilliert. Die Temperatur auf dem 2. theoretischen Boden von unten wurde auf 90 bis 100°C gehalten. Während der Umsetzung wurden weitere 20 g 3-Methyl-butan-2-on so zudosiert, dass das Reaktionsgemisch bei 160°C immer am Sieden war. Nach beendeter Zugabe des Acetessigsäuremethylesters wurde das Reaktionsgemisch noch für 1 Stunde auf 165°C gehalten, bis kein $CO_2$ mehr gebildet wurde. Geranylaceton wurde bei quantitativem Umsatz an Acetessigsäuremethylester mit einer Selektivität von 94,2%, bezogen auf Linalool und 87,2%, bezogen auf Acetessigsäuremethylester, erhalten.

B. Vergleichsversuch

Arbeitet man wie unter A beschrieben, nur ohne den Zusatz von 3-Methyl-butyn-2-on, so war es nach beendetem Zutropfen von Acetessigsäuremethylester notwendig, das Reaktionsgemisch noch für 12 Stunden nachzuheizen, bis die $CO_2$-Entwicklung zum Stehen kam. Geranylaceton wurde hierbei bei einem Umsatz von 97% des Acetessigsäuremethylesters in einer Selektivität von 87%, bezogen auf Linalool, und 80,2%, bezogen auf Acetessigsäuremethylester, erhalten.

*Beispiel 2*

Herstellung von 6,10-Dimethyl-5-undecen-2-on

In der in Beispiel 1 beschriebenen Apparatur wurde eine Mischun aus 624 g (4 Mol) 3,7-Dimethyl-1-octen-3-ol, 8,5 g Aluminium-triisopropylat sowie 70 g Pentan-3-on (Siedetemperatur 101,7°C) auf 165°C erhitzt. Anschliessend wurden innerhalb von 4 Stunden 464 g (4 Mol) Acetessigsäuremethylester zugetropft. Die Temperatur auf dem 2. theoretischen Boden von unten wurde dabei auf 100 bis 110°C gehalten. Während der Umsetzung wurden weitere 20 g Pentan-3-on so zudosiert, dass das Reaktionsgemisch bei 165°C immer am Sieden war. Nach Zugabe des Acetessigsäuremethylesters wurde das Reaktionsgemisch noch nachgeheizt bis die $CO_2$-Entwicklung beendet war, wozu etwa 1 Stunde benötigt wurde. Die Selektivität an 6,10-Dimethyl-5-undecen-2-on beträgt bei quantitativem Umsatz des Acetessigsäuremethylesters 96,5%, bezogen auf 3,7-Dimethyl-1-octen-3-ol und 91,1%, bezogen auf Acetessigsäuremethylester.

*Vergleichsversuch*

(Umsetzung in Gegenwart hochsiedender Lösungsmittel)

In der in Beispiel 1 beschriebenen Apparatur wurde eine Mischung aus 624 g (4 Mol) 3,7-Dimethyl-1-octen-3-ol, 18,5 g Al-isopropylat und 180 ml Isophoron (Kp = 215 bis 216°C) vorgelegt und hierzu innerhalb von 6 Stunden 464 g (4 Mol) Acetessigsäuremethylester zugetropft. Das hierbei entstehende Methanol und $CO_2$ wurden über Kopf der Kolonne abgezogen. Nach Zulaufende waren noch 22% unumgesetztes 3,7-Dimethyl-1-octen-3-ol im Kolben vorhanden.

Das Reaktionsgemisch wurde daher noch 4 Stunden (bis sich kein $CO_2$ mehr bildete) bei 165°C gehalten. Die Konzentration an Ausgangsverbindung ist dann auf 7% abgefallen. Durch Aufdestillation des Reaktionsaustrages wurden 577 g 6,10-Dimethyl-5-undecen-2-on erhalten. Bei einem Umsatz von etwa 90% entspricht dies einer Selektivität von 89%, bezogen auf 3,7-Dimethyl-1-octen-3-ol bzw. 79%, bezogen auf Acetessigsäuremethylester.

*Beispiel 3*

Herstellung von Farnesylaceton

A. In der in Beispiel 1 beschriebenen Apparatur wurde eine Mischung aus 888 g (4 Mol) Nerolidol, 10 g Aluminium-tri-sek.-butylat sowie 100 g 3-Methyl-butan-2-on auf 165°C erhitzt. Anschliessend wurde zu dieser Mischung innerhalb von 3 Stunden 464 g (4 Mol) Acetessigsäuremethylester zugetropft. Die Temperatur auf dem 2. theoretischen Boden wurde auf 90 bis 100°C gehalten. Während der Umsetzung wurden weitere 30 g 3-Methyl-butan-2--on so zudosiert, dass das Reaktionsgemisch bei 165°C immer am Sieden war. Nach Zugabe des Acetessigsäuremethylesters wurde das Reaktionsgemisch noch für 1 Stunde nachgeheizt bis kein $CO_2$ mehr gebildet wurde. Farnesylaceton wurde bei quantitativem Umsatz des Acetessigsäuremethylesters in einer Selektivität von 93,2%, bezogen auf Nerolidon und 85,0%, bezogen auf Acetessigsäuremethylester, erhalten.

B. Vergleichsversuch

Arbeitet man wie unter A beschrieben, jedoch ohne Zusatz von 3-Methyl-butyn-2-on, so war es nach beendetem Zutropfen von Acetessigsäuremethylester notwendig, das Reaktionsgemisch noch für 8 Stunden nachzuheizen bis die $CO_2$-Entwicklung zum Stehen kam. Farnesylaceton wurde hierbei in einer Selektivität von 80,8%, bezogen auf Nerolidol und 72,7%, bezogen auf Acetessigsäuremethylester, erhalten. Der Umsatz an Acetessigsäuremethylester betrug 98%.

*Beispiel 4*

Herstellung von 6,10,14-Trimethyl-5-pentadecen--2-on

In der in Beispiel 1 beschriebenen Apparatur wurde ein Gemisch aus 904 g (4 Mol) 3,7,11-Trimethyl-1--dodecen-3-ol, 8,5 g Aluminium-triisopropylat und 100 g Pentan-3-on auf 165°C erhitzt. Zu diesem Gemisch wurden innerhalb von 4 Stunden 464 g Acetessigsäuremethylester zugetropft. Die Temperatur auf dem 2. theoretischen Boden von unten wurde auf 100 bis 110°C gehalten. Während der Umsetzung wurden weitere 25 g Pentan-3-on so zudosiert, dass das Reaktionsgemisch bei 165°C immer am Sieden war. Nach der Zugabe des Acetessigsäuremethylesters wurde das Reaktionsgemisch noch für 1 Stunde nachgeheizt bis die $CO_2$-Entwicklung beendet war. Die Selektivität an 6,10,14-Trimethyl-5-pentadecen-2-on betrug bei quantitativem Umsatz des Acetessigsäuremethylesters 96,3%, bezogen auf 3,7,11-Trimethyl-1-dodecen-3-ol und 91,0%, bezogen auf Acetessigester.

*Beispiele 5 bis 11*

Als Apparatur wurde ein 4-l-Kolben mit aufgesetzter Kolonne sowie zwei Tropftrichtern verwendet. Die Kolonne wies etwa 20 bis 30 theoretische Böden auf. Die Temperatur konnte sowohl in der Destillationsblase als auch jeweils alle 4 bis 5 theoretische Böden von unten bis zum Kopf der Kolonne gemessen werden.

Die Temperaturregelung der Apparatur erfolgte so, dass bei konstanter Heizleistung (d.h. etwa konstanter Ölbadtemperatur) die Reaktionstemperatur im Sumpf durch entsprechend dosierte Zugabe des Zwischensieders geregelt wurde. Der Ablauf am Kopf der Kolonne wurde über die Temperatur auf dem 8. theoretischen Boden von unten geregelt. Diese Temperatur wurde so eingestellt, dass die Temperatur am 4. theoretischen Boden von unten der Siedetemperatur des Zwischensieders bei Normaldruck und die Temperatur am Kopf der Kolonne etwa der Siedetemperatur des Methanols entsprach. Letztere liegt zunächst wegen des Partialdrucks des abgespaltenen $CO_2$ etwas unterhalb von 65°C.

Die Reaktion wurde so gefahren, dass der ungesättigte Alkohol zusammen mit dem als Katalysator verwendeten Aluminium-triisopropylat vorgelegt wurde und dann in einer bestimmten Zeit $t_z$, z.B. in 4 Stunden, der Acetessigsäuremethylester (AEE) zugetropft wurde. Danach sind noch 1 bis höchstens 2 Stunden Nachreaktionszeit $t_N$ erforderlich, um quantitativen Umsatz an AEE und an dem als Zwischenprodukt auftretenden Allyl-acetoacetat zu erreichen, was am einfachsten daran zu erkrnnen ist, dass kein $CO_2$ mehr gebildet wird. Da während dieser Nachheizzeit ohnehin kaum noch Methanol anfällt, wurde die Sumpftemperatur über die Heizung aufrechterhalten, wodurch es in der letzten halben Stunde zu einem «Zusammenfallen» des Kolonnenprofils kam.

In der folgenden Tabelle werden die Reaktionsbedingungen und Ergebnisse der Versuche zusammengefasst:

Die in der Tabelle benutzten Abkürzungen haben folgende Bedeutung:

AEE = Acetessigsäuremethylester
HLIN = 3,7-Dimethyl-1-octen-3-ol (Dihydrolinalool)
HDHL = 3,7-Dimethyl-1-octin-3-ol
MDHL = 3,7-Dimethyl-7-methoxy-1-octin-3-ol
$t_z + t_N$ = Zugabezeit + Nachreaktionszeit

Das in der Tabelle angegebene Molverhältnis der Ausgangsstoffe, des Alkohols II und des AEE, wurde «telquel» berechnet. Hierbei kompensierten sich dann zwei Effekte: Der eingesetzte AEE hatte eine Reinheit von ~ 99%, der eingesetzte Alkohol wies jedoch nur eine Reinheit von 93 bis 97% auf. Da aber die Ausbeute bezüglich AEE etwas schlechter ist, vom Alkohol blieben etwa 2 bis 3% übrig. Nach Beendigung des AEE-Zulaufs betrug die AEE-Konzentration etwa 2 bis 3 Gew.-%.

Die Selektivitäten beziehen sich jedoch nicht auf die eingesetzten Alkohole II «telquel», sondern sind umgerechnet auf 100%ige Ware, um eine bessere Vergleichbarkeit der Versuche untereinander zu ermöglichen.

| Nr. | Alkohol II | Molverh. Alkohol II/AEE | Al-isoprop. [Mol%] bez. Alkohol II | Zwischensieder | Reaktionszeit $t_Z + t_N$ [h] | Kolonnentemperatur *) [°C] | Reaktionstemperatur [°C] | Selektivität bez. AEE [%] | Selektivität bez. Alkohol II [%] |
|---|---|---|---|---|---|---|---|---|---|
| 5 | HLIN | 1:1 | 1,0 | 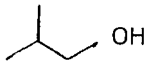 | 6 + 7 | 105 | 165 | 76,8 | 95,7 |
| 6 | HLIN | 1:1 | 1,0 | 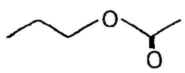 | 6 + 3 | 90 | 165 | 86,1 | 94,8 |
| 7 | HLIN | 1:1 | 1,0 | 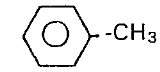 | 6 + 2 | 100 | 165 | 86,5 | 92,7 |
| 8 | HLIN | 1:1 | 1,0 | 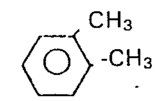 | 6 + 2 | 130 | 165 | 87,0 | 91,5 |
| 9 | HLIN | 1:1 | 1,0 | 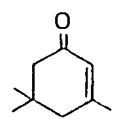 | 4 + 8 | 60 | 165 | 83,2 | 89,3 |
| 10 | HDHL | 1:1 | 1,0 | 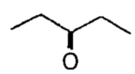 | 5 + 6 | 100 | 175 | 60,8 | 70,2 |
| 11 | MDHL | 1:1 | 1,0 | 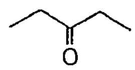 | 5 + 6 | 100 | 175 | 29,2 | 38,2 |

*) Temperatur in der Kolonne am 2. theoretischen Boden von unten

*Beispiel 12*

Kombinierte Herstellung von 6,10-Dimethyl-5-undecen-2-on und 2-Methyl-2-hepten-6-on

Als Apparatur wurde ein 2-l-Kolben mit aufgesetzter Fraktionierkolonne verwendet, die etwa 20 theoretische Böden aufwies. Die Kolonne war in regelmässigen Abständen mit Thermometern bestückt, so dass ausser am Kopf und im Sumpf der Kolonne etwa an jedem 4. theoretischen Boden die Temperatur gemessen werden kann.

Eine Mischung aus 637 g 3,7-Dimethyl-1-octen-3-ol, 8,6 g Al-Isopropylat, 36 g Acetessigsäuremethylester und 78 g 3-Methyl-1-buten-3-ol wurde auf 165°C erhitzt und hierzu innerhalb von 5 Stunden 642 g Acetessigsäuremethylester und 161 g 3-Methyl-1-buten-3-ol zugetropft. Die Temperatur auf dem 2. theoretischen Boden wurde durch entsprechendes Einstellen des Rücklaufverhältnisses auf 90 bis 100°C gehalten.

Entstehendes Methanol und $CO_2$ wurde über Kopf auf der Kolonne abdestilliert.

Nach beendeter Zugabe der Komponenten wurde das Reaktionsgemisch noch 3 Stunden lang bei 165°C gehalten bis die $CO_2$-Entwicklung beendet war. Der eingesetzte Acetessigsäuremethylester ist dann quantitativ umgesetzt. Durch destillative Aufarbeitung wurden 701 g 6,10-Dimethyl-5-undecen-2-on und 216 g 2-Methyl-2-hepten-6-on erhalten.

Dies entspricht einer Selektivität von 95,5%, bezogen auf 3,7-Dimethyl-1-octen-3-ol, von 94%, bezogen auf 3-Methyl-1-buten-3-ol und von 90%, bezogen auf den Acetessigsäuremethylester.

*Beispiel 13*

Kombinierte Herstellung von 6,10,14-Trimethyl-5-pentadecen-2-on und 2-Methyl-2-hepten-6-on

In der in Beispiel 12 beschriebenen Apparatur wurden eine Mischung aus 637 g 3,7,11-Trimethyl-1-dodecen-3-ol (86%ig), 8,6 g Al-Isopropylat und 60 g 3-Methyl-1-buten-3-ol auf 165°C erhitzt und hierzu innerhalb von 5 Stunden 471 g Acetessigsäuremethylester und 159 g 3-Methyl-1-buten-3-ol zugetropft, wobei die Temperatur auf den 2. theoretischen Boden der Kolonne durch entsprechendes Einstellen des Rücklaufverhältnisses auf etwa 90 bis 100°C gehalten wurde. Nach einer Nachreaktionszeit von 2 Stunden bei 165°C wurde destillativ aufgearbeitet. Es wurden 598 g 6,10,14-Trimethyl-5-pentadecen-2-on und 161 g 2-Methyl-2-hepten-6-on erhalten.

Das entspricht einer Selektivität von etwa 94%, bezogen auf 3,7,11-Trimethyl-1-dodecen-3-ol, von etwa 96%, bezogen auf 3-Methyl-1-buten-3-ol und von 87%, bezogen auf den Acetessigsäuremethylester.

*Beispiel 14*

Kombinierte Herstellung von Geranylaceton und 2-Methyl-2-hepten-6-on

In der in Beispiel 12 beschriebenen Apparatur wurde eine Mischung aus 637 g Linalool (91%ig), 36 g Acetessigsäuremethylester, 6 g 3-Methyl-1-buten-3-ol und 8,6 g Aluminium-isopropylat auf 165°C erhitzt und hierzu innerhalb von 10 Stunden unter Aufrechterhalten einer Reaktionstemperatur von 165°C 642 g Acetessigsäuremethylester und 211 g 3-Methyl-1-buten-3-ol zugetropft, wobei die Temperatur auf dem 2. theoretischen Boden auf etwa 100°C gehalten wurde. Nach einer Nachreaktionszeit von 4 Stunden bei 165°C wurde destillativ aufgearbeitet. Man erhielt 681 g Geranylaceton, 212 g 2-Methyl-2-hepten-6-on und 37 g unumgesetztes 3-Methyl-1-buten-3-ol. Die erzielten Selektivitäten haben demnach folgende Werte: 87%, bezogen auf 3-Methyl-1-buten-3-ol; 94%, bezogen auf Linalool und 88%, bezogen auf Acetessigsäuremethylester.

*Beispiel 15*

Kontinuierliche Herstellung von Geranylaceton

Für die kontinuierliche Umsetzung bestand das Reaktorsystem aus einer «Kessel-Kaskade», bestehend aus 3 miteinander durch einen Überlauf verbundenen Kolben 1 bis 3 mit einem Inhalt von je einem Liter, die in der folgenden Zeichnung schematisch wiedergegeben ist. Die Brüden der Reaktionsgefässe wurden zusammen in die untere Haube der Kolonne 4 mit etwa 20 theoretischen Böden geführt. Der Frischzulauf 5, bestehend aus einem Gemisch von 1 Mol Acetessigsäuremethylester, 1 Mol Linalool und 2,1 g Aluminium-tri-iso-propylat wurde mit einer Geschwindigkeit von 300 ml/Stunde in den ersten Kolben gegeben. Auch der Zwischensieder 6, hier 3-Methyl-1-propan-2-on, wurde beim Anfahren der Reaktion in den ersten Kolben gegeben. Methanol und $CO_2$ wurden über Kopf der Kolonne entnommen. Der Kolonnenablauf vom untersten theoretischen Boden der Kolonne wurde aufgeteilt und in den ersten und zweiten Kolben zurückgeleitet. In diesen Rückstrom wurde je nach Bedarf als Zwischensieder 6 das 3-Methyl-propan-2-on gegeben, so dass eine Sumpftemperatur von 165°C in allen 3 Kolben bei gutem Sieden eingehalten werden konnte. Es stellt sich eine Konzentration von 4 bis 6 Gew.% an 3-Methyl-butan-2-on (gaschromatographisch bestimmt) in den Kolben ein.

Die Temperatur auf dem 2. theoretischen Boden von unten der Kolonne wurde auf 90 bis 100°C gehalten. Hierbei wurde bei einem Umsatz von etwa 99,9%, bezogen auf Acetessigsäuremethylester, eine Selektivität an Geranylaceton von 95,1%, bezogen auf Linalool und von 89,2%, bezogen auf Acetessigsäuremethylester, erzielt.

**Patentansprüche**

1. Verfahren zur Herstellung von ungesättigten Ketonen der allgemeinen Formel I

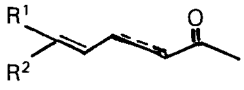

(I)

in der die gestrichelte Linie eine zusätzliche C-C-Bindung bedeuten kann, $R^1$ für eine Alkylgruppe mit 1 bis 4 C-Atomen steht und $R^2$ für eine gesättigte oder ungesättigte Alkyl-, Cycloalkyl- oder Cycloalkylalkylgruppe mit 4 bis 30 C-Atomen steht, oder für eine Gruppe der allgemeinen Formel

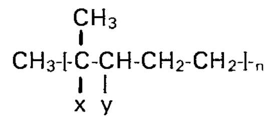

steht, in der n für eine ganze Zahl von 1 bis 6 steht und x und y entweder beide für H stehen, oder x für Methoxy und y für H stehen, oder aber x und y zusammen eine zusätzliche Bindung zwischen den x und y tragenden C-Atomen bedeuten, durch Umsetzen von ungesättigten Alkoholen der allgemeinen Formel II

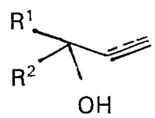

(II)

mit Acetessigsäurealkylestern der allgemeinen Formel III

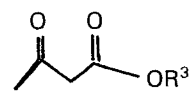

(III)

in der $R^3$ für Alkyl mit 1 bis 4 C-Atomen steht, bei erhöhter Temperatur in Gegenwart von 0,5 bis 5 Gew.-%, bezogen auf den umzusetzenden Acetessigsäurealkylester, an einer organischen Aluminiumverbindung der allgemeinen Formel

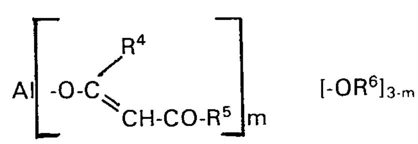

in der $R^4$ und $R^5$ Alkyl- oder Alkoxygruppen mit 1 bis 4 C-Atomen, vorzugsweise Methyl- oder Äthylgruppen bedeuten, $R^6$ für eine Alkylgruppe mit 1 bis 4 C-Atomen steht und m jeweils für die Zahlen 0, 1, 2 oder 3 steht, oder einem Aluminiumtriaryloxylat unter Abspaltung und fortlaufender destillativer Entfernung eines Alkohols der allgemeinen Formel IV

$$R^3 - OH \quad (IV)$$

in einem Reaktorsystem mit aufgesetzter Fraktionierkolonne, dadurch gekennzeichnet, dass man

A. von ungesättigten Alkoholen der Formel II ausgeht, deren Siedepunkte höher liegen als die der verwendeten Acetessigsäurealkylester;

B. dass man die Umsetzung in Gegenwart von 0,5 bis 100 Gew.%, bezogen auf den Alkohol der Formel II, einer Flüssigkeit (im folgenden als «Zwischensieder» bezeichnet) durchführt, die sich unter den Reaktionsbedingungen inert verhält und deren Siedepunkt zwischen dem des eingesetzten Acetessigesters der allgemeinen Formel III und dem des hieraus abzuspaltenden Alkohols der allgemeinen Formel IV liegt, oder aber dass man die Umsetzung in Gegenwart von 3-Methyl-1-buten-3-ol als Zwischensieder durchführt, wobei zusätzlich als erwünschte Nebenreaktion dessen teilweise Umsetzung mit dem Acetessigsäurealkylester zu 2-Methyl-2-hepten-6-on als weiterem Wertprodukt stattfindet, und

C. dass die Temperatur am unteren Ende der Fraktionierkolonne nicht mehr als 40°C höher ist als der Siedepunkt des Zwischensieders beim jeweils verwendeten Druck.

2. Verfahren gemäss Anspruch 1, dadurch gekennzeichnet, dass man die Umsetzung in Gegenwart von einem Keton mit 4 bis 7 Kohlenstoffatomen als Zwischensieder, der sich unter den Reaktionsbedingungen inert verhält, durchführt.

3. Verfahren gemäss Anspruch 1, dadurch gekennzeichnet, dass die Temperatur am unteren Ende der Fraktionierkolonne nicht mehr als 20°C höher ist als der Siedepunkt des Zwischensieders beim jeweils verwendeten Druck.

4. Verfahren gemäss Anspruch 1, dadurch gekennzeichnet, dass man als Acetessigsäurealkylester entweder Acetessigsäuremethylester, Acetessigsäureäthylester oder Acetessigsäureisopropylester verwendet.

5. Verfahren gemäss Anspruch 1, dadurch gekennzeichnet, dass man als ungesättigten Alkohol der allgemeinen Formel II einen solchen verwendet, in dem $R^1$ für $CH_3$ und $R^2$ für eine Gruppe der allgemeinen Formel

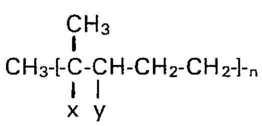

steht, in der n für eine ganze Zahl von 1 bis 6 steht und x und y entweder beide für H stehen, oder x für Methoxy und y für H stehen, oder aber x und y zusammen eine zusätzliche Bindung zwischen den x und y tragenden C-Atomen bedeuten.

6. Verfahren gemäss Anspruch 1, dadurch gekennzeichnet, dass man die Umsetzung in Gegenwart von 3-Methyl-1-buten-3-ol als Zwischensieder durchführt, wobei zusätzlich als erwünschte Nebenreaktion dessen teilweise Umsetzung mit dem Acetessigsäurealkylester zu 2-Methyl-2-hepten-6-on als weiterem Wertprodukt stattfindet.

**Claims**

1. A process for the preparation of unsaturated ketones of the general formula I

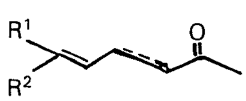

(I)

where the broken line may denote an additional C-C bond, $R^1$ is alkyl of 1 to 4 carbon atoms and $R^2$ is saturated or unsaturated alkyl, cycloalkyl or cycloalkylalkyl of 4 to 30 carbon atoms, or a group of the general formula

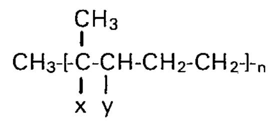

where n is an integer from 1 to 6 and x and y are both H, or x is methoxy and y is H, or x and y together are an additional bond between the carbon atoms carrying x and y by reacting an unsaturated alcohol of the general formula II

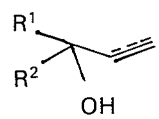

(II)

with an alkyl acetoacetate of the general formula III

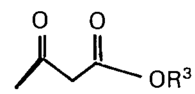

(III)

where $R^3$ is alkyl of 1 to 4 carbon atoms, at an elevated temperature in the presence of from 0.5 to 5% by weight, based on the alkyl acetacetate to be reacted, of an organic aluminium compound of the general formula

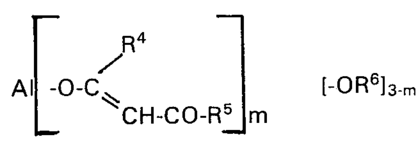

where $R^4$ and $R^5$ are alkyl or alkoxy of 1 to 4 carbon atoms, preferably methyl or ethyl, $R^6$ is alkyl of 1 to 4 carbon atoms and n is 0, 1, 2 or 3, or an aluminium triaryloxylate, with elimination and continuous distillative removal of an alcohol of the general formula IV

$$R^3 - OH \qquad (IV)$$

in a reactor system carrying a fractionating column, wherein

A. the unsaturated alcohol of the formula II which is used as starting compound has a boiling point higher than that of the alkyl acetoacetate used,

B. the reaction is carried out in the presence of from 0.5 to 100% by weight, based on the alcohol of the formula II, of a liquid (referred to hereinafter as «intermediate-boiling liquid») which is inert under the reaction conditions and which has a boiling point between that of the alkyl acetoacetate starting material of the general formula III and the alcohol of the general formula IV to be eliminated therefrom, or the reaction is carried out in the presence of 3-methyl-but-1-en-3-ol as intermediate-boiling liquid, the partial reaction thereof with the alkyl acetoacetate additionally taking place, as a desired side-reaction giving 2-methyl-hept-2-en-6-one as an additional useful product, and

C. the temperature at the bottom of the fractionating column is not more than 40°C higher than the boiling point of the intermediate-boiling liquid, under the prevailing pressure.

2. A process as claimed in claim 1, wherein the reaction is carried out in the presence of a ketone of 4 to 7 carbon atoms as the intermediate-boiling liquid which is inert under the reaction conditions.

3. A process as claimed in claim 1, wherein the temperature at the bottom of the fractionating column is not more than 20°C higher than the boiling point of the intermediate-boiling liquid, under the prevailing pressure.

4. A process as claimed in claim 1, wherein the alkyl acetoacetate used is methyl acetoacetate, ethyl acetoacetate or isopropyl acetoacetate.

5. A process as claimed in claim 1, wherein the unsaturated alcohol of the general formula II is a compound where $R^1$ is $CH_3$ and $R^2$ is a group of the general formula

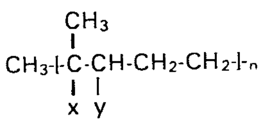

where n is an integer from 1 to 6 and x and y are both H, or x is methoxy and y is H, or x and y together are an additional bond between the carbon atoms carrying x and y.

6. A process as claimed in claim 1, wherein the reaction is carried out in the presence of 3-methyl-but-1-en-3-ol as intermediate-boiling liquid, the partial reaction thereof with the alkyl acetoacetate additionally taking place, as a desired side-reaction, giving 2-methyl-hept-2-en-6-one as an additional useful product.

**Revendications**

1. Procédé pour la préparation de cétones insaturées de formule générale I

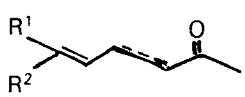

(I)

dans laquelle la ligne de pointillés peut signifier une liaison C-C supplémentaire, $R^1$ represente un groupe alcoyle à 1-4 atomes de C et $R^2$ un groupe alcoyle, cycloalcoyle ou cycloalcoylalcoyle saturé ou insaturé à 4-30 atomes de C ou groupe de formule générale

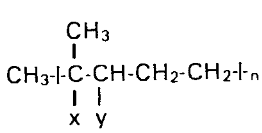

dans laquelle n est un nombre entier de 1 à 6, et x et y représentent l'un et l'autre H, ou x est un methoxy et y H, ou encore x et y représentent ensemble une liaison supplémentaire entre les atomes de C qui portent x et y, par la mise en réaction d'alcools insaturés de formule générale II

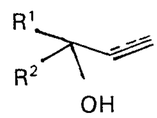

(II)

avec des acétylacétates d'alcoyle de formule générale III

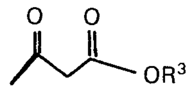

(III)

dans laquelle $R^3$ est un alcoyle à 1-4 atomes de carbone, à température élevée, en présence de 0,5 à 5% en poids, par rapport à l'acétylacétate d'alcoyle mis en réaction, d'un composé organique de l'aluminium de formule générale

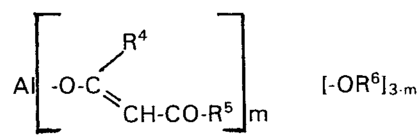

dans laquelle $R^4$ et $R^5$ représentent des groupes alcoyle ou alcoxy à 1-4 atomes de C, de préférence des groupes méthyle ou éthyle, $R^6$ représente un groupe alcoyle à 1-4 atomes de C et m est l'un des nombres 0, 1, 2 et 3, ou un triaryloxylate d'aluminium, avec séparation et élimination continue par distillation d'un alcool de formule générale IV

$$R^3 - OH \qquad (IV)$$

dans un système de réacteur surmonté d'une colonne de fractionnement, caractérisé

A. en ce qu'on part d'alcools insaturés de formule II dont le point de fusion se situe plus haut que celui de l'acétylacétate d'alcoyle utilisé;

B. en ce qu'on mène la réaction en présence de 0,5 à 100% en poids, par rapport à l'alcool de formule II, d'un liquide (ci-après appelé «liquide de point d'ébullition intermédiaire») qui a un comportement inerte dans les conditions de la réaction et dont le point d'ébullition se situe entre celui de l'acétylacétate de formule générale III utilisé et celui de l'alcool qui doit se former par séparation à partir de celui-ci, ou bien on mène la réaction en présence de 3-méthyl-1-butène-3-ol servant de liquide de point d'ébullition intermédiaire, auquel cas il se produit en plus, en tant que réaction secondaire désirable, la réaction partielle de celui-ci avec l'acétylacétate d'alcoyle pour donner la 2-méthyl-2-heptène-6-one qui est un produit de valeur supplémentaire, et

C. en ce que la température à l'extrémité inférieure de la colonne de fractionnement n'est pas supérieure de plus de 40°C au point d'ébullition du liquide de point d'ébullition intermédiaire, à la pression particulière appliquée.

2. Procédé selon la revendication 1, caractérisé en ce qu'on mène la réaction en présence d'une cétone à 4-7 atomes de carbone en tant que liquide de point d'ébullition intermédiaire qui a un comportement inerte dans les conditions de la réaction.

3. Procédé selon la revendication 1, caractérisé en ce que la température à l'extrémité inférieure de la colonne de fractionnement n'est pas supérieure de plus de 20 °C au point d'ébullition du liquide de point d'ébullition intermédiaire, à la pression particulière appliquée.

4. Procédé selon la revendication 1, caractérisé en ce qu'on utilise, en tant qu'acétylacétate d'alcoyle, soit l'acétylacétate de méthyle, soit l'acétylacétate d'éthyle, soit l'acétylacétate d'isopropyle.

5. Procédé selon la revendication 1, caractérisé en ce qu'on utilise, en tant qu'alcool insaturé de formule générale II, un alcool dans lequel $R^1$ représente $CH_3$, et $R^2$ un groupe de formule générale

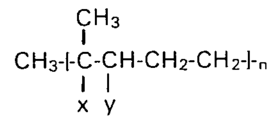

dans laquelle n est un nombre entier de 1 à 6, et x et y représentent l'un et l'autre H, ou x est un methoxy et y H, ou encore x et y représentent ensemble une liaison supplémentaire entre les atomes de C qui portent x et y.

6. Procédé selon la revendication 1, caractérisé en ce qu'on mène la réaction en présence de 3-méthyl-1-butène-3-ol servant de liquide de point d'ébullition intermédiaire, auquel cas il se produit en plus, en tant que réaction secondaire désirable, la réaction partielle de celui-ci avec l'acétylacétate d'alcoyle pour donner la 2-méthyl-2-heptène-6-one qui est un produit de valeur supplémentaire.

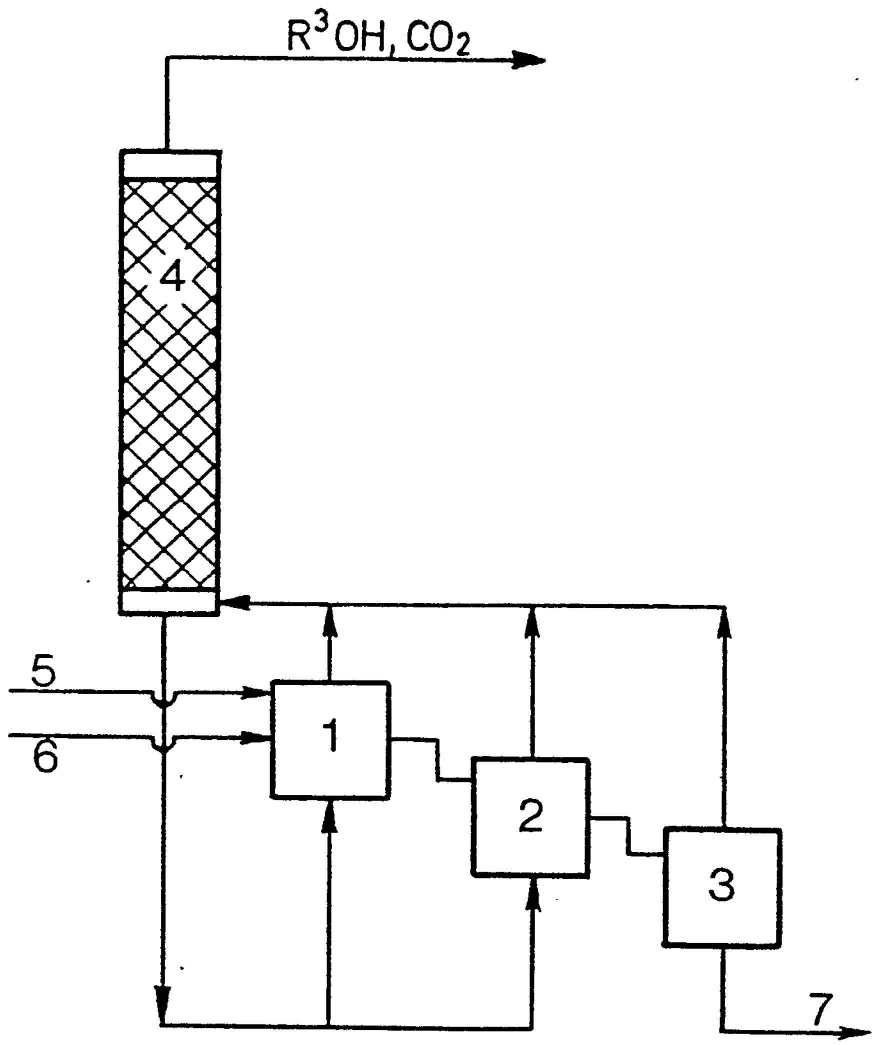
$R^3OH, CO_2$
4
5
6
1
2
3
7